# EUROPEAN PATENT APPLICATION

(11) **EP 4 385 407 A1**
(43) Date of publication of application: **19.06.2024**
(21) Application number: 22916236.7
(22) Date of filing: 02.06.2022
(51) Int. Cl.: A61B 5/1495, A61B 5/145, A61B 5/155, A61B 5/00

(54) **CORRECTION ALARM METHOD**

(30) Priority: 29.12.2021 KR 20210190646
(71) Applicant: i-Sens, Inc., Seoul 06646 (KR)
(72) Inventor: NAH, Ji Seon, Seoul 06646 (KR); SEO, Jung Hee, Seoul 06646 (KR); JANG, Min Ji, Seoul 06646 (KR); KANG, Yun Hee, Seoul 06646 (KR); KANG, Young Jea, Seoul 06646 (KR)
(74) Representative: BCKIP Part mbB
(86) International application number: PCT/KR2022/007877
(87) International publication number: WO 2023/128081

(57) **Abstract**

The present invention relates to a method for providing a correction alarm in a continuous biometric information measurement system and, more particularly, to a correction alarm method comprising: calculating the next correction time on the basis of an actual correction time of a user, thereby informing the user of the next correction time calculated on the basis of the actual correction time even if correction is performed differently from that of a preset correction period; and dividing a total use period of time of a sensor into a plurality of correction sections and differently calculating the next correction time on the basis of a correction section, to which a time at which an actual reference biometric value is input belongs, from among the plurality of correction sections, so as to use a reference biometric value input at every correction time, thereby accurately correcting a biometric value.

## Description

### TECHNICAL FIELD

The present disclosure relates to a method of providing a calibration alarm in a biometric information measurement system, and more specifically, related to a calibration alarm method in which, by calculating the next calibration time based on the actual calibration time of a user, even if calibration is performed differently from the preset calibration cycle, the next calibration time can be calculated based on the actual calibration time and notified to a user, and by dividing the entire usage period of the sensor into multiple calibration periods and calculating the next calibration time differently based on the calibration period to which the time at which the reference biometric value is input belongs among the plurality of calibration periods, the biometric value can be accurately calibrated using the reference biometric value entered at each calibration time.

### BACKGROUND

Diabetes is a chronic medical condition that is common in modern people, and in the Republic of Korea, there are 2 million diabetes patients, about 5% of the total population.

Diabetes occurs when the absolute level of the sugar level in blood is high due to the absolute deficiency or relative insufficiency of insulin, produced by the pancreas, caused by various reasons such as obesity, stress, poor eating habits, and inherited hereditary factors and imbalance regarding glucose in the blood.

The blood usually contains a certain concentration of glucose, and tissue cells gain energy from the glucose.

However, when the glucose is increased excessively more than needed, the glucose cannot be properly stored in the liver, muscle, or adipose tissue and is accumulated in the blood, because of this, patients with diabetes maintain a much higher blood glucose level than normal people, and as excessive blood glucose passes through the tissues and is discharged into the urine, it results in deficiency of glucose, which is absolutely necessary for all tissues of the body, thereby causing abnormalities in respective body tissues.

Diabetes is characterized by substantial absence of subjective symptoms at the beginning of the condition, when diabetes progresses, diabetes-specific symptoms such as overdrink, overeat, polyuria, weight loss, weariness, skin itchiness, and lower ability of naturally healing on injury on hands and feet are shown, and further progression of diabetes leads to complications such as visual disturbances, hypertension, kidney disease, paralysis, periodontal disease, muscle spasms and neuralgia, as well as gangrene.

In order to diagnose diabetes beforehand and manage to prevent the progression of diabetes into complications associated therewith, systematic blood glucose measurement and treatment should be performed.

Diabetes needs to constantly measure blood glucose for management, so the demand for devices related to blood glucose measurement is steadily increasing. It has been confirmed through various studies that, when diabetic patients strictly control the management of blood glucose, the incidence of complications of diabetes is significantly reduced. Accordingly, it is very important for diabetic patients to measure blood glucose regularly for blood glucose management.

The finger prick method is generally used to manage blood glucose level in diabetic patients, and although such a blood collecting glucose measurement device helps diabetics patients manage blood glucose level, it is difficult to accurately identify frequently changing blood glucose levels because only the results at the time of measurement appear. In addition, the finger prick method requires blood collection to measure blood glucose levels frequently throughout the day, which poses a significant burden on diabetic patients.

Diabetics patients generally experience hyperglycemia and hypoglycemia, and an emergency may occur in the hypoglycemic conditions. Hypoglycemia occurs when sugar content is not kept for a long time, and the patients may become unconscious or die in the worst case. Accordingly, rapid discovery of the hypoglycemic condition is critically important for diabetics. The blood-gathering-type biometric information measuring device intermittently measuring glucose has limited ability to accurately measure blood glucose levels.

Recently, to overcome such a drawback of the blood-collecting-type biometric information measuring device, continuous glucose monitoring systems (CGMSs) inserted into the human body to measure a blood glucose level every few minutes have been developed, and therefore easily perform the management of diabetics and responses to an emergency situation.

The continuous blood glucose monitoring system includes a sensor transmitter that measures biometric values from body fluids by being attached to the body of a user, and a communication terminal that outputs information about the received biometric values to a user. The sensor transmitter includes a sensor for continuous blood glucose measurement that is partially inserted into the human body, and the sensor is inserted into the human body for a certain period of use, for example, approximately 15 days. The sensor transmitter periodically measures biometric values from body fluids, and a biometric management application is installed in the communication terminal to periodically receive biometric values from the sensor transmitter and output the information about the received biometric values to a user.

The sensor of the sensor transmitter is continuously inserted into a skin throughout a usage period, and the sensitivity of the sensor may vary depending on the part of the body where the sensor is inserted, and even if the sensor insertion location on the body part is the same, the sensitivity of the sensor may change over time. Biometric values measured by a sensor transmitter have errors according to changes in sensitivity, and the biometric values of a user must be calibrated by applying calibration factor to the measured biometric values to overcome the errors.

In order to provide accurate biometric values to a user, the biometric values received from the sensor transmitter must be initially calibrated and then continuously calibrated at certain calibration intervals during the usage period of the sensor transmitter. More specifically, during initial calibration, the initial calibration factor is calculated from the reference biometric value measured through a separate measuring device, such as a blood- collecting glucose measurement device and the biometric value received from the sensor transmitter, and then the received biometric values are calibrated by applying an initial calibration factor to the received biometric values.

During the usage period of the sensor transmitter, the calibration factor is calculated from the reference biometric values measured by a separate measuring device and the biometric values received from the sensor transmitter at each calibration cycle, and the received biometric values must be calibrated by applying the calibration factor until the next calibration cycle arrives.

It takes a certain amount of time for the sensor to stabilize after the sensor is inserted into a human body, and in order to accurately measure biometric values before the sensor is stabilized, more frequent calibration is required than after the sensor is stabilized. Therefore, the communication terminal provides a calibration alarm to a user when a set calibration cycle arrives, thereby encouraging a user to input reference biometric values at each set calibration cycle.

However, in a case in which a user inputs the reference biometric values at any time regardless of the set calibration cycle, since calibration alarms are provided to a user again at a preset calibration cycle regardless of the actual input time of the reference biometric value, a user has the problem of repeating unnecessary calibration or receiving unnecessary calibration alarms. Meanwhile, even if a user inputs reference biometric values regardless of the preset calibration cycle, a calibration alarm is provided to a user at the preset calibration cycle, so there is a problem in that the biometric values cannot be accurately calibrated because the difference between the time of inputting the reference biometric values and the next calibration time is outside the appropriate calibration interval.

### SUMMARY

### Technical Problem

As the present disclosure is intended to solve the problems of the conventional calibration alarm method mentioned above, the purpose of the present disclosure is to provide a calibration alarm method that calculate the next calibration time based on the actual calibration time of a user, calculate the next calibration time, and then provide a calibration alarm to a user based on the calculated calibration time.

Another purpose of the present disclosure is to provide a calibration alarm method that divide the entire usage period of the sensor into a plurality of calibration periods and accurately calibrate biometric values by calculating the next calibration time differently based on the calibration period to which the actual input time of the reference biometric value belongs among a plurality of calibration periods.

Another purpose of the present disclosure is to provide a calibration alarm method that can prevent unnecessary calibration alarms from being provided to a user by calculating the next calibration time based on the period to which the actual input time of the reference biometric value belongs, regardless of the preset calibration cycle.

### Solution to Problem

To accomplish the above-described purposes, according to an embodiment of the present disclosure, a calibration alarm method may comprise: determining an input time of a reference biometric value for calibrating a measurement biometric value measured by a sensor; determining a calibration period in which the input time of the reference biometric value is included; calculating a next calibration time based on the determined calibration period; and providing a calibration alarm to a user based on the calculated next calibration time, wherein the sensor is configured to be insertable into body of the user and measure biometric information of the user for a certain period of time.

Here, the sensor is a sensor configured to measure a blood glucose level of the user, and the reference biometric value is a reference blood glucose value measured using a separate blood glucose meter to calibrate a measurement blood glucose value measured by the sensor.

The calibration alarm method according to an embodiment of the present disclosure may, when a next reference biometric value is input regardless of the next calibration time, recalculate a calibration time based on a calibration period in which an input time of the next reference biometric value is included.

The calibration alarm method according to an embodiment of the present disclosure may calculate a calibration factor using the reference biometric value or the next reference biometric value, and, until a new reference biometric value is input, calibrate the measurement biometric value using the calibration factor.

In the calibration alarm method according to an embodiment of the present disclosure, the calibration period is divided into an additional stabilization period, a buffer period, and a final stabilization period.

In the calibration alarm method according to an embodiment of the present disclosure, when the input time of the reference biometric value is within the additional stabilization period, the next calibration time is calculated based on which one calibration period among the additional stabilization period, the buffer period, and final stabilization period includes a first calibration cycle preset after the input time of the reference biometric value.

In the calibration alarm method according to an embodiment of the present disclosure, if the first calibration cycle preset after the input time of the reference biometric value is within the additional stabilization period, the next calibration time is calculated using the first calibration cycle preset after the input time of the reference biometric value.

In the calibration alarm method according to an embodiment of the present disclosure, if the first calibration cycle preset after the input time of the reference biometric value is within the buffer period, the next calibration time is calculated using the first calibration cycle preset after the input time of the reference biometric value.

In the calibration alarm method according to an embodiment of the present disclosure, if the first calibration cycle preset after the input time of the reference biometric value is within the final stabilization period, the next calibration time is calculated using an end time point of the buffer period.

In the calibration alarm method according to an embodiment of the present disclosure, if the input time of the reference biometric value is within the buffer period or the final stabilization period, the next calibration time is calculated using a second calibration cycle preset after the input time of the reference biometric value.

To accomplish the above-described purposes, according to an embodiment of the present disclosure, a continuous biometric information measurement system may comprise: a sensor configured to be insertable into body of a user and configured to continuously measure a biometric value of the user for a certain period of time; and a user terminal configured to receive the measured biometric value and calibrate the measured biometric value using a reference biometric value, wherein the user terminal is configured to determine an input time of the reference biometric value for calibrating the measured biometric value measured by the sensor, determine a calibration period in which the input time of the reference biometric value is included, calculate a next calibration time based on the determined calibration period and provide a calibration alarm to the user based on the calculated next calibration time.

### Advantageous Effects of Invention

The calibration alarm method according to the present disclosure has the following effects.

First, the calibration alarm method according to the present disclosure calculates a next calibration time based on an actual calibration time of a user, so that even if calibration is performed differently from a preset calibration cycle, the next calibration time calculated based on the actual calibration time can be informed to a user.

Second, by calculating the next calibration time differently based on a calibration period to which the actual input time of the reference biometric values belongs among multiple calibration periods, the biometric value can be accurately calibrated using the reference biometric value entered at each calibration time.

Third, the calibration alarm method according to the present disclosure calculates the next calibration time based on a period in which the actual input time of a reference biometric value belongs, regardless of a preset calibration cycle, thereby preventing unnecessary calibration alarms from being provided to a user.

### BRIEF DESCRIPTION OF THE DRAWINGS

FIG. 1 is a schematic diagram showing a biometric value measurement system according to an embodiment of the present disclosure.
FIG. 2 is a diagram for explaining an example of inputting initial calibration information or periodic calibration information.
FIG. 3 is a functional block diagram for explaining a calibration alarm device according to the present disclosure.
FIG. 4 is a functional block diagram for explaining a calibration notification unit according to the present disclosure.
FIG. 5 is a flowchart for explaining a calibration alarm method according to the present disclosure.
FIG. 6 is a diagram for explaining an example of an entire calibration period used in the calibration alarm method according to the present disclosure.
FIG. 7 is a flowchart illustrating an example of calculating a next calibration time according to the present disclosure.
FIG. 8 is a diagram illustrating an example of calculating a next calibration time when an input time of a reference blood glucose value falls within an additional stabilization period.
FIG. 9 is a diagram illustrating an example of calculating a next calibration time when an input time of a reference blood glucose value does not fall within an additional stabilization period.
FIG. 10 is a diagram for explaining an example of a method for providing a calibration alarm in the present disclosure.
FIG. 11 i shows an example of a calibration alarm provided from the input control unit (133) by the user interface.
FIG. 12 shows an example of a calibration alarm provided from the input control unit (133) by the user interface.

### DETAILED DESCRIPTION OF EMBODIMENTS

It should be noted that the technical terms used in the present disclosure are only used to describe specific embodiments and are not intended to limit the present disclosure. In addition, the technical terms used in the present disclosure, unless specifically defined in a different sense in the present disclosure, should be interpreted as meanings generally understood by those skilled in the art in the technical field to which the present disclosure belongs, and should not be interpreted as an excessively comprehensive sense or an excessively reduced sense. In addition, if the technical term used in the present disclosure is an incorrect technical term that does not accurately express the idea of the present disclosure, it should be replaced with a technical term that can be correctly understood by a person skilled in the art.

Additionally, as used in the present disclosure, singular expressions include plural expressions unless the context clearly dictates otherwise. In the present disclosure, terms such as "consists of" or "comprises" should not be construed as necessarily including all of the various components or steps described in the disclosure and should be interpreted that some of the components or steps may not be included, or additional components or steps may be included.

In addition, it should be noted that the attached drawings are only intended to facilitate easy understanding of the concepts of the present disclosure, and should not be construed as limiting the concepts of the present disclosure by the attached drawings.

Hereinafter, the calibration alarm method according to the present disclosure will be examined in more detail with reference to the attached drawings.

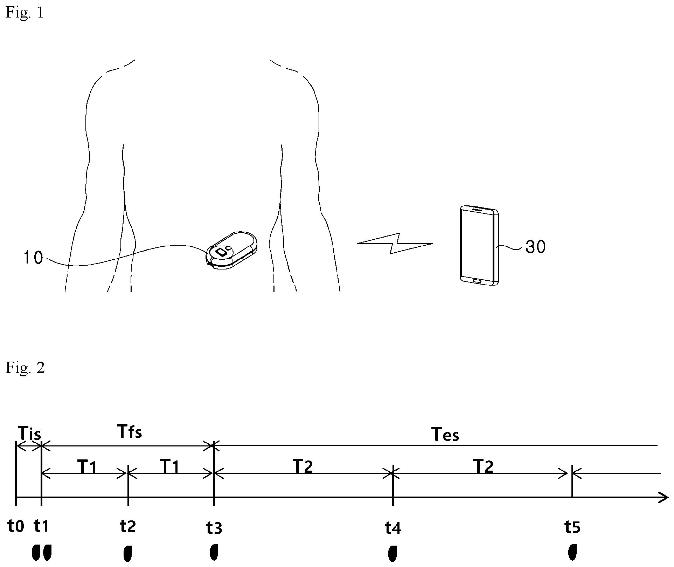

FIG. 1 is a schematic diagram showing a biometric value measurement system according to an embodiment of the present disclosure.

Hereinafter, a blood glucose value will be described as an example of a biometric value, and a reference blood glucose value will be described as an example of a reference biometric value, but various biometric values other than a blood glucose value can be measured depending on the field to which the present disclosure is applied.

Referring to FIG. 1, a biometric value measurement system (1) according to an embodiment of the present disclosure includes a sensor transmitter (10) and a communication terminal (30).

The sensor transmitter (10) is attached to a body, and when the sensor transmitter (10) is attached to the body, one end of the sensor of the sensor transmitter (10) is inserted into a skin and periodically measures a blood glucose signal indicating a blood glucose value from the body fluids of a human body during the usage period of the sensor.

The communication terminal (30) is a terminal that may receive a blood glucose signal from the sensor transmitter (10), calibrate the received blood glucose signal with a calibration factor, convert the unit into a calibrated blood glucose value, and then display it to a user, so a terminal capable of communicating with the sensor transmitter (10), such as a smartphone, tablet PC, or laptop, may be used. Of course, the communication terminal (30) is not limited thereto, and may be any type of terminal may be used as long as it includes a communication function and a program or application may be installed.

That is, the sensor transmitter (10) generates a blood glucose signal, for example, a current signal, corresponding to the blood glucose value of a user and transmits the blood glucose signal to the communication terminal (30), and the communication terminal (30) calibrates the blood glucose signal of the current value with a calibration factor and converts the unit into a calibrated blood glucose value. Depending on the field to which the present disclosure is applied, the sensor transmitter (10) can directly convert units from a blood glucose signal to a blood glucose value, and the communication terminal (30) can calibrate the blood glucose value received from the sensor transmitter (10) with a calibration factor.

Hereinafter, in the present disclosure, the blood glucose signal or the unit-converted blood glucose value measured by the sensor transmitter (10) is referred to as a measured blood glucose value, and the blood glucose value obtained by calibrating the blood glucose signal or the unit-converted blood glucose value with a calibration factor in the communication terminal (30) is referred to as the calibrated blood glucose value.

The sensor transmitter (10) transmits information about the measured blood glucose value to the communication terminal (30) at the request of the communication terminal (30) or at a set time, and for data communication between the sensor transmitter (10) and the communication terminal (30), the sensor transmitter (10) and the communication terminal (30) may be connected to each other through a wired communication method such as a USB cable or a wireless communication method such as infrared communication, NFC communication, or Bluetooth.

Looking more specifically, when communication is connected between the sensor transmitter (10) and the communication terminal (30), the initial calibration factor is calculated using the reference blood glucose value measured by a separate blood glucose meter (not shown) after stabilization of the sensor transmitter (10), and initial calibration is performed on the measured blood glucose value using the initial calibration factor. Thereafter, the communication terminal (30) calibrates the measured blood glucose value received from the sensor transmitter (10) with an initial calibration factor, and provides the calibrated blood glucose value to a user.

In order to accurately calibrate the blood glucose value measured by the sensor transmitter (10), the communication terminal (30) calculates a new calibration factor using the reference blood glucose value measured periodically by a separate blood glucose meter during the usage period of the sensor transmitter (10), and calculates the calibrated blood glucose value by calibrating the measured blood glucose value received from the sensor transmitter using the new calibration factor, and then outputs the calculated calibrated blood glucose value to a user.

FIG. 2 is a diagram to explain an example of inputting initial calibration information and additional calibration information, wherein the calibration information is the reference blood glucose value of a user measured by a test strip through a separate blood glucose meter. Referring to FIG. 2, a sensor transmitter is initially stabilized from the time point when the sensor transmitter and a communication terminal are connected (t0) until the set initial stabilization time (TiS) elapses.

At the time when the sensor of the sensor transmitter is initially stabilized (t1), initial calibration information is input to the communication terminal. Here, the initial calibration information may be entered multiple times to accurately calculate a calibration factor. The communication terminal calculates the initial calibration factor using the initial calibration information and the blood glucose value measured by the sensor transmitter, and calculates a calibrated blood glucose value of a user by calibrating the measured blood glucose value received from the sensor transmitter using the initial calibration factor.

After the sensor of the sensor transmitter is initially stabilized, new calibration information is additionally input to the communication terminal preferably at a calibration cycle of 12 hours, 24 hours, etc., until the expiration of the usage period of the sensor transmitter, and after the sensor of a sensor transmitter has initially stabilized, the sensor may require additional stabilization for a certain period of time, and in an additional stabilization period (Tfs), new calibration information can be input at a calibration cycle (T1) of every 12 hours, and in a final stabilization period (Tes) after the additional stabilization period, new calibration information can be input to the sensor of the sensor transmitter at a calibration cycle (T2) of 24 hours, 48, etc.

When a sensor is manufactured in the same environment and under the same conditions, the sensor has a certain sensitivity change characteristic (sensitivity drift) after insertion into a human body, and based on the sensitivity change characteristic (sensitivity drift), depending on the manufacturing environment of the sensor, the length of the additional stabilization period or the final stabilization period may be set differently, or the calibration cycle in the additional stabilization period or the final stabilization period may be set differently.

Whenever new calibration information is input, the communication terminal calculates a new calibration factor to be used to calibrate the measured blood glucose value received by the sensor transmitter from the time when the new calibration information is input, and calculates a blood glucose value of a user by calibrating the measured blood glucose value received from the sensor transmitter using the new calibration factor.

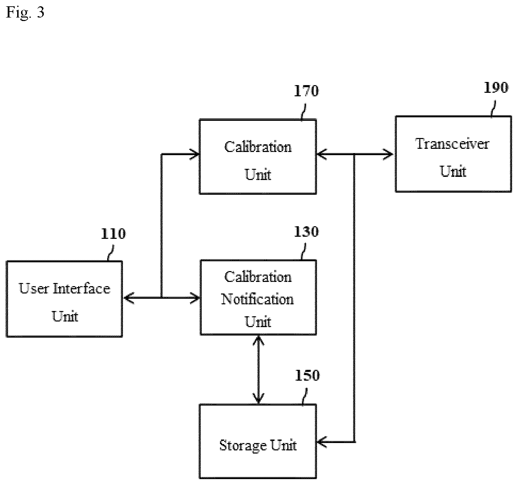

FIG. 3 is a functional block diagram for explaining a calibration alarm device according to the present disclosure.

The calibration alarm device may be implemented in a communication terminal such as a smart terminal that communicates with the sensor transmitter and notifies a user of a calibrated blood glucose value, or may be implemented through a separate receiving device.

Looking more specifically with reference to FIG. 3, in a case in which a user command for inputting a reference blood glucose value is input through a user interface unit (110), a calibration notification unit (130) activates an input screen for inputting the reference blood glucose value on the user interface unit (110), and a user may input the reference blood glucose value through the activated input screen.

Here, the calibration notification unit (130) determines whether a calibration cycle has arrived based on a calibration cycle stored in a storage unit (150), and when the calibration cycle has arrived, a calibration alarm may be provided to a user through the user interface unit (110), and a user inputs a reference blood glucose value based on the calibration alarm. However, depending on the field to which the present disclosure is applied, a reference blood glucose value can be entered at any time during the entire calibration period at the request of a user, regardless of a calibration cycle.

When a user inputs a reference blood glucose value regardless of a calibration cycle, the calibration alarm unit (130) determines a calibration period to which an input time belongs based on the input time of a reference blood glucose value, and a calibration cycle is recalculated based on the determined calibration period. The calibration cycle stored in the storage unit (150) may be updated with the recalculated calibration cycle, and the calibration alarm unit (150) provides a calibration alarm to a user through the user interface unit (110) when a next calibration time arrives based on a updated calibration cycle.

When a reference blood glucose value is input, a calibration unit (170) determines a measured blood glucose value corresponding to the input time of a reference blood glucose value among the measured blood glucose values received through a transceiver unit (190), and generates a calibration pair consisting of a reference blood glucose value and a corresponding measured blood glucose value. The calibration unit (170) calculates a new calibration factor using the calibration pair. The calibration unit (170) calculates a calibrated blood glucose value by calibrating a measured blood glucose value received after a reference blood glucose value is input using a new calibration factor, and provides the calculated calibrated blood glucose value to a user through the user interface unit (110).

Depending on the field to which the present disclosure is applied, the calibration unit (170) may calculate the calibration factor using the current calibration pair consisting of the input reference blood glucose value and the corresponding measured blood glucose value, but in order to calculate the accurate calibration factor, a new calibration factor may be calculated with a regressive method using the current calibration pair and the past calibration pairs.

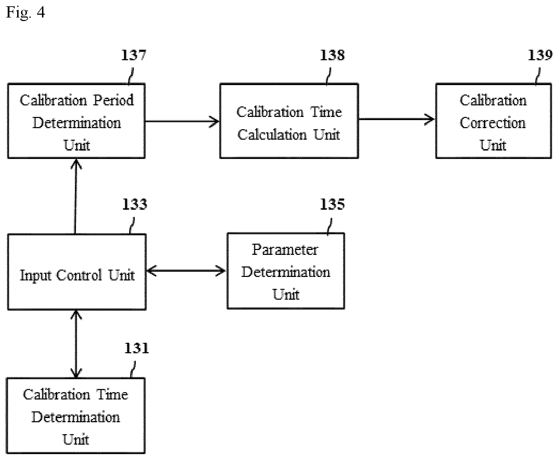

FIG. 4 is a functional block diagram for explaining the calibration notification unit according to the present disclosure.

Looking more specifically with reference to FIG. 4, a calibration time determination unit (131) determines whether the calibration cycle has arrived based on the calibration cycle stored in the storage unit, and provides a notification signal for notifying that the calibration period has arrived to an input control unit (133) when the calibration cycle has arrived.

The input control unit (133) activates the input screen on the user interface unit based on a notification signal or an input command for a reference blood glucose value input through the user interface unit regardless of the notification signal, and when the reference blood glucose value is input through the input screen, a calibration period determination unit (137) determines the calibration period to which the reference blood glucose value input time belongs in the entire calibration period of the sensor based on the reference blood glucose value input time.

The entire calibration period of the sensor can be sequentially divided into an additional stabilization period, a buffer period, and a final stabilization period based on the time point of the completion of initial stabilization after the sensor is inserted into the skin.

A calibration time calculation unit (138) calculates a next calibration time based on the calibration period to which the input time of a reference blood glucose value belongs, calculates the next calibration time previously stored in the storage unit, and then updates a calibration time.

Preferably, the calibration notification unit according to the present disclosure may further include a parameter determination unit (135), and when a next calibration time arrives, the input control unit (133) determines whether a calibration condition parameter such as a rate of change in a calibration blood glucose value or a size of a calibration blood glucose value satisfies a calibration condition before providing a calibration alarm to a user or at the same time providing a calibration alarm to a user. If the calibration condition parameter satisfies the calibration condition, the input control unit (133) may activate an input screen on the user interface screen to allow a user to input a reference blood glucose value.

Preferably, the calibration notification unit according to the present disclosure may further include a calibration correction unit (139), and the calibration alarm can be canceled according to a user command so that the calibration alarm is not provided at the next calibration time, or the next calibration time can be adjusted based on user schedule information, or the next calibration time can be adjust to an adjacent time of the next calibration time, such as 1 hour before, 2 hours before, 1 hour after, 2 hours after the next calibration time, etc.

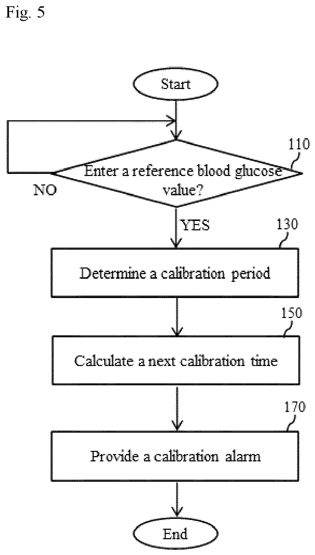

FIG. 5 is a flowchart for explaining the calibration alarm method according to the present disclosure.

Looking more specifically with reference to FIG. 5, it is determined whether a reference blood glucose value has been entered (S110). When a reference blood glucose value is input, it is determined which calibration period the input time of the reference blood glucose value belongs to in an entire calibration period (S130).

Here, the entire calibration period can be sequentially divided into an additional stabilization period, a buffer period, and a final stabilization period in chronological order, starting from the completion of initial stabilization. According to the field to which the present disclosure is applied, the entire calibration period can be divided into various calibration periods, which fall within the scope of the present disclosure.

Here, a reference blood glucose value can be input in a calibration cycle according to a user command, or can be input at any time in the entire calibration period regardless of the calibration cycle.

A next calibration time is calculated based on a calibration period to which the input time of a reference blood glucose value belongs, that is, the next calibration time is calculated based on which calibration period among the additional stabilization period, the buffer period, and the final stabilization period (S 150). If the next calibration time is calculated, information about the next calibration time can be provided in advance at the time when the next calibration time is calculated, even before the next calibration time arrives.

Meanwhile, when the next calibration time arrives, a calibration alarm is generated, and the generated calibration alarm is provided to a user (S170).

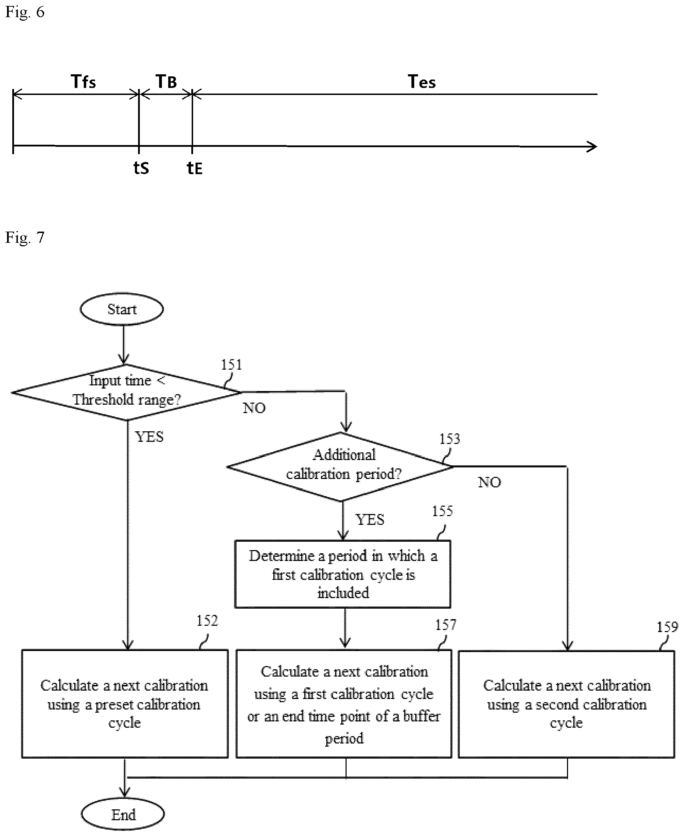

FIG. 6 is a diagram for explaining an example of the entire calibration period used in the calibration alarm method according to the present disclosure.

As shown in FIG. 6, after the initial stabilization time point (11), it can be sequentially divided into an additional stabilization period (Tfs), a buffer period (TB), and a final stabilization period (Tes) in chronological order. The additional stabilization period (Tfs) is a period that requires further stabilization of the sensor after the initial stabilization but before the final stabilization period (Tes), and in the additional stabilization period (Tfs), the reference blood glucose value is input and calibrated every first calibration cycle, and and in the final stabilization period (Tes), the reference blood glucose value is input and calibrated every second calibration cycle. Here, the first calibration cycle is characterized as being equal to or shorter than the second calibration cycle. In this way, by receiving and calibrating the reference blood glucose value more frequently in the additional stabilization period (Tfs) than in the final stabilization period (Tes), the sensor can accurately calibrate the measured blood glucose value until final stabilization is achieved.

FIG. 7 is a flowchart illustrating an example of calculating the next calibration time according to the present disclosure.

Looking more specifically with reference to FIG. 7, it is determined whether the input time of the reference blood glucose value is within a threshold range from the preset calibration cycle (S151). If the input time of the reference blood glucose value falls within a threshold range from the preset calibration cycle, a calibration factor is generated using the input reference blood glucose value, and the next calibration time is calculated using the preset calibration cycle (S152).

However, if the input time of the reference blood glucose value is outside a threshold range compared to the preset calibration cycle, it is determined whether the input time of the reference blood glucose value falls within the additional stabilization period (S153). If the input time of the reference blood glucose value is within an additional stabilization period, the first calibration cycle is added to the input time of the reference blood glucose value to determine a calibration period to which the first calibration cycle after the input time of the reference blood glucose value belongs (S155). Depending on whether the calibration period to which the first calibration cycle after the input time of the reference blood glucose value belongs is an additional stabilization period, a buffer period, or a final stabilization period, the next calibration time is calculated using the first calibration cycle or the end time point of the buffer period (S157).

However, if the input time of the reference blood glucose value is not in the additional stabilization period, that is, if the input time of the reference blood glucose value belongs to the buffer period or the final stabilization period, the next calibration time is calculated using the second calibration cycle (S159).

Depending on the field to which the present disclosure is applied, the step of determining whether the input time of the reference blood glucose value is within a threshold range from the preset calibration cycle is omitted, and it is only determined whether the input time of the reference blood glucose value belongs to any calibration period among the additional stabilization period, buffer period, or final stabilization period, and based on this, the next calibration time may be calculated based on the input time of the reference blood glucose value in the manner described above.

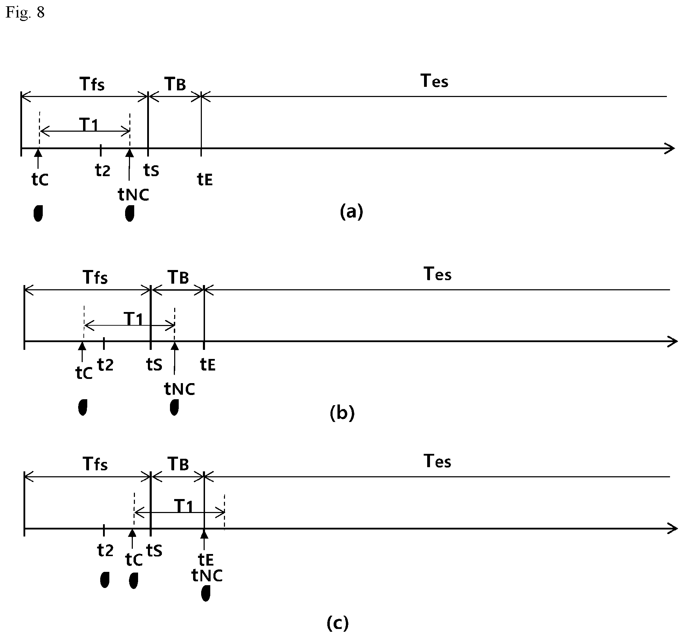

FIG. 8 is a diagram illustrating an example of calculating the next calibration time in a case in which the input time of the reference blood glucose value falls within the additional stabilization period.

As shown in FIG. 8(a), if a first calibration cycle (T₁) preset after the input time of the reference biometric value (tc) falls within the additional stabilization period (T_{fs}), the next calibration time (t_{NC}) is recalculated using the first calibration cycle (T₁) preset after the input time of the reference biometric value (tc) .

As shown in FIG. 8(b), in a case in which the first calibration cycle (T₁) preset after the input time of the reference biometric value (tc) falls within the buffer period (T_{B}), the next calibration time (t_{NC})is recalculated using the first calibration cycle (T₁) preset after the input time of the reference biometric value (tc).

As shown in FIG. 8(c), in a case in which the first calibration cycle (T₁) preset after the input time of the reference biometric value (tc) falls within the final stabilization period (Tₑₛ), the next calibration time (t_{NC}) is recalculated using the end time point (t_{E}) of the buffer period.

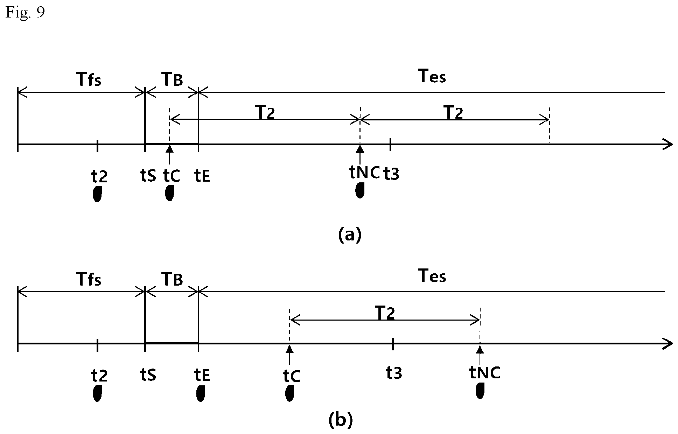

FIG. 9 is a diagram illustrating an example of calculating the next calibration time when the input time of the reference blood glucose value does not belong to the additional stabilization period.

As shown in FIG. 9(a), when the input time of the reference biometric value (tc) falls within the buffer period (T_{B}), the next calibration time (t_{NC}) is recalculated using a second calibration cycle (T₂) preset after the input time of the reference biometric value (tc).

As shown in FIG. 9(b), when the input time of the reference biometric value (tc) falls within the final stabilization period (Tₑₛ), the next calibration time (t_{NC}) is recalculated using the second calibration cycle (T₂) preset after the input time of the reference biometric value (tc).

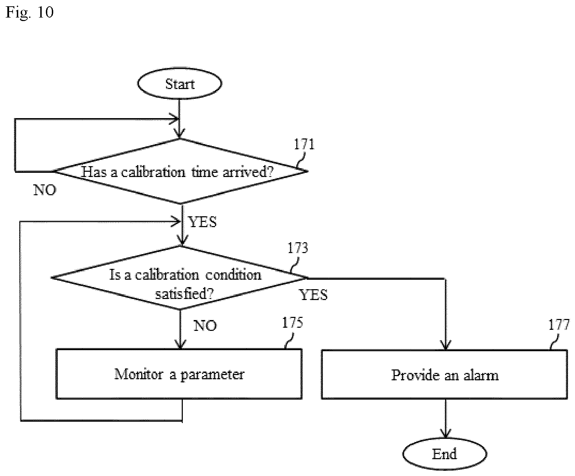

FIG. 10 is a diagram for explaining an example of a method for providing a calibration alarm in the present disclosure.

Looking more specifically with reference to FIG. 10, it is determined whether the next calibration time has arrived (S171).

When the next calibration time arrives, it is determined whether the calibration condition parameter measured at the time when the next calibration time arrives satisfies the calibration condition (S173). Here, the calibration condition parameter may be a rate of change of the calibrated blood glucose value, a lower limit threshold value, or an upper limit threshold value. In this way, by determining whether the calibration condition parameter satisfies the calibration condition before or when providing a calibration alarm, it prevents the calibration factor from being calculated using the reference blood glucose value measured at a time when the rate of change of blood glucose value exceeds threshold rate, indicating a rapid rise or fall in the blood glucose value. Or, by determining whether the calibration condition parameter satisfies the calibration condition before or when providing the calibration alarm, it prevents the calibration factor from being calculated using the reference blood glucose value measured at the time when the blood glucose value exceeds the lower or upper threshold value.

If the calibration condition parameter measured at the time when the next calibration time arrives satisfies the calibration condition, a calibration alarm is generated and the generated calibration alarm is provided to a user (S177).

Meanwhile, if the calibration condition parameter measured at the time when the next calibration time arrives does not satisfy the calibration condition, the calibration condition parameter is monitored for a certain period of time after the next calibration time (S175) to determine whether the calibration condition parameter satisfies the calibration condition within a certain period of time. If the calibration condition parameter satisfies the calibration condition within a certain period of time, a calibration alarm is generated and provided to a user.

However, if the calibration condition parameter does not satisfy the calibration condition after a certain period of time, an error message is generated and the generated error message is provided to a user. Here, the error message may be a message to remove the sensor, a message to calibrate by entering a plurality of reference blood glucose values, or a message that calibration is not possible, depending on the error condition.

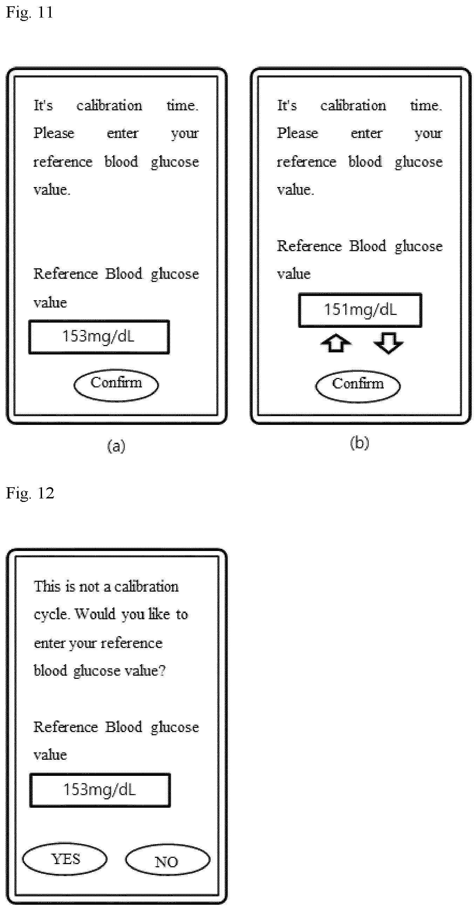

FIG. 11 shows an example of a calibration alarm provided by the input control unit (133) through the user interface.

As shown in FIG. 11(a), the calibration alarm is activated with a screen for entering a reference blood glucose value along with a message notifying that it is the calibration time, and a user can measure the reference blood glucose value using a separate blood glucose meter and sensor strip, and calculate a calibration factor by directly entering the measured reference blood glucose value into the input screen.

However, if a user directly inputs the reference blood glucose value like this, when entering the reference blood glucose value, a user may make a mistake and enter a value that is different from the actual reference blood glucose value as the reference blood glucose value. In this case, there is a problem in that the measured blood glucose value cannot be accurately calibrated. In addition, when measuring the reference blood glucose value, if the hands of a user are contaminated with sugar, etc., the reference blood glucose value may be measured as a value different from the actual blood glucose value, and in this case, there is a problem in that the measured blood glucose value cannot be accurately calibrated due to the incorrectly measured reference blood glucose value.

As shown in FIG. 11(b), when providing a calibration alarm, the input control unit (133) provides the current calibrated blood glucose value calculated based on the blood glucose value measured through the sensor on the input screen. As the current calibrated blood glucose value is a blood glucose value calculated with a calibration factor which is calculated immediately before the current calibration cycle, the value does not significantly deviate from the actual blood glucose value of a user, and a user can start from the current calibrated blood glucose value provided on the input screen and input the reference blood glucose value using an icon (e.g. arrows) to increase or decrease the difference between the measured reference blood glucose value and the current calibrated blood glucose value. In this way, by allowing the reference blood glucose value to be entered based on the current calibrated blood glucose value, it is possible to prevent a user from accidentally entering a different reference blood glucose value, and if the difference between the measured reference blood glucose value and the current calibrated blood glucose value is large, a user can be encouraged to measure the reference blood glucose value again.

FIG. 12 shows an example of a calibration alarm provided by the input control unit (133) through the user interface.

As shown in FIG. 12, when a calibration command for inputting a reference blood glucose value is input through the user interface unit, the next calibration time and the time at which the calibration command is input are compared, and if the calibration command is entered at a time different from the next calibration time, a screen for inputting the reference blood glucose value is activated along with a message informing a user that the calibration cycle has not arrived.

In this way, when a calibration command to input a reference blood glucose value is entered regardless of the calibration cycle, the reference blood glucose value can be entered at any time regardless of the calibration cycle, but by informing a user that the current calibration cycle has not arrived, a user can be prevented from collecting blood and entering the reference blood glucose unnecessarily.

Meanwhile, the above-described embodiments of the present disclosure can be written as a program that can be executed on a computer, and can be implemented in a general-purpose digital computer that operates the program using a computer-readable recording media.

The computer-readable recording media include storage media such as magnetic storage media (e.g., ROM, floppy disk, hard disk, etc.), optical reading media (e.g., CD-ROM, DVD, etc.), and carrier wave (e.g., transmission via Internet).

The present disclosure has been described with reference to the embodiments shown in the drawings, but these are merely examples, and those skilled in the art will understand that various modifications and other equivalent embodiments are possible therefrom. Therefore, the true scope of technical protection of the present disclosure should be determined by the technical concepts of the attached registration claims.

## Claims

1. A calibration alarm method comprising:
determining an input time of a reference biometric value for calibrating a measurement biometric value measured by a sensor;
determining a calibration period in which the input time of the reference biometric value is included;
calculating a next calibration time based on the determined calibration period; and
providing a calibration alarm to a user based on the calculated next calibration time,
wherein the sensor is configured to be insertable into body of the user and measure biometric information of the user for a certain period of time.

2. The calibration alarm method according to claim 1, wherein:
the sensor is a sensor configured to measure a blood glucose level of the user, and
the reference biometric value is a reference blood glucose value measured using a separate blood glucose meter to calibrate a measurement blood glucose value measured by the sensor.

3. The calibration alarm method according to claim 1,
wherein when a next reference biometric value is input regardless of the next calibration time, a calibration time is recalculated based on a calibration period in which an input time of the next reference biometric value is included.

4. The calibration alarm method according to claim 3,
wherein the calibration alarm method calculates a calibration factor using the reference biometric value or the next reference biometric value, and, until a new reference biometric value is input, calibrates the measurement biometric value using the calibration factor.

5. The calibration alarm method according to claim 4,
wherein, in the calibration alarm method, the calibration period is divided into an additional stabilization period, a buffer period, and a final stabilization period.

6. The calibration alarm method according to claim 5,
wherein when the input time of the reference biometric value is within the additional stabilization period, the next calibration time is calculated based on which one calibration period among the additional stabilization period, the buffer period, and final stabilization period includes a first calibration cycle preset after the input time of the reference biometric value.

7. The calibration alarm method according to claim 6,
wherein if the first calibration cycle preset after the input time of the reference biometric value is within the additional stabilization period, the next calibration time is calculated using the first calibration cycle preset after the input time of the reference biometric value.

8. The calibration alarm method according to claim 6,
wherein if the first calibration cycle preset after the input time of the reference biometric value is within the buffer period, the next calibration time is calculated using the first calibration cycle preset after the input time of the reference biometric value.

9. The calibration alarm method according to claim 6,
wherein if the first calibration cycle preset after the input time of the reference biometric value is within the final stabilization period, the next calibration time is calculated using an end time point of the buffer period.

10. The calibration alarm method according to claim 5,
wherein if the input time of the reference biometric value is within the buffer period or the final stabilization period, the next calibration time is calculated using a second calibration cycle preset after the input time of the reference biometric value.

11. The calibration alarm method according to claim 1, wherein the providing of the calibration alarm comprises:
determining whether the next calibration time has arrived;
determining whether a calibration condition parameter measured at a time point when the next calibration time arrives satisfies a calibration condition; and
if the calibration condition parameter satisfies the calibration condition, generating the calibration alarm and providing the generated calibration alarm to the user.

12. The calibration alarm method according to claim 11,
wherein if the calibration condition parameter measured at the time point when the next calibration time arrives does not satisfy the calibration condition, the calibration condition parameter is monitored for a set time, and if the calibration condition parameter monitored for the set time satisfies the calibration condition, the calibration alarm is generated.

13. A continuous biometric information measurement system, comprising:
a sensor configured to be insertable into body of a user and configured to continuously measure a biometric value of the user for a certain period of time; and
a user terminal configured to receive the measured biometric value and calibrate the measured biometric value using a reference biometric value,
wherein the user terminal is configured to determine an input time of the reference biometric value for calibrating the measured biometric value measured by the sensor, determine a calibration period in which the input time of the reference biometric value is included, calculate a next calibration time based on the determined calibration period and provide a calibration alarm to the user based on the calculated next calibration time.
